# EUROPÄISCHE PATENTSCHRIFT

(11) **EP 1 791 774 B1**
(45) Veröffentlichungstag und Bekanntmachung des Hinweises auf die Patenterteilung: **17.06.2009**
(21) Anmeldenummer: 05790827.9
(22) Anmeldetag: 19.09.2005
(51) Int. Cl.: B65G 65/48

(54) **SELBSTENTLEERENDER BEHÄLTER UND VERFAHREN ZUR ENTLEERUNG EINES BEHÄLTERS**
SELF-EMPTYING CONTAINER AND METHOD FOR EMPTYING A CONTAINER
CONTENANT A AUTO-EVACUATION ET PROCEDE POUR VIDER UN CONTENANT

(30) Priorität: 20.09.2004 DE 102004045847
(43) Veröffentlichungstag der Anmeldung: 06.06.2007
(73) Patentinhaber: Kraemer, Thilo, 64291 Darmstadt (DE)
(72) Erfinder: Kraemer, Thilo, 64291 Darmstadt (DE)
(74) Vertreter: Mierswa, Klaus
(86) Internationale Anmeldenummer: PCT/DE2005/001646
(87) Internationale Veröffentlichungsnummer: WO 2006/032247

(56) Entgegenhaltungen:
- DE-A1- 1 955 419
- US-A- 5 305 912

## Beschreibung

### Technisches Gebiet:

Die Erfindung betrifft einen selbstentleerenden Behälter und ein Verfahren zur Entleerung eines Behälters.

### Stand der Technik:

Zum Stand der Technik ist durch die DE-1955419-A eine Vorrichtung zum gleichmäßigen Verteilen von Stoffen ungleichmäßiger Beschaffenheit bekannt geworden, insbesondere von klebrigen, feucht gewordenen oder klumpigen Stoffen, durch die Zentrifugalwirkung einer am Boden eines Behälters rotierenden Verteilerscheibe. Die Verteilerscheibe ist als Schnecke mit Abstreifschaufeln ausgebildet, wobei auf der Schnecke eine glatte Scheibe von geringerem Durchmesser als diese und mit dieser mitdrehend befestigt ist und senkrecht über der Scheibe die Unterseite eines in dem Behälter angeordneten Trichters liegt. Unterhalb des Trichters ist eine ringförmige Kammer vorgesehen, aus der der jetzt gleichmäßig verteilte Stoff durch eine Abzugsöffnung austritt.

### Technische Aufgabe:

Der Erfindung liegt die Aufgabe zu Grunde, einen selbstentleerenden Behälter und ein Verfahren zur Entleerung eines Behälters anzugeben, wobei der selbstentleerende Behälter z.B. in einer Vorrichtung zur Prüfung von physikalischen oder chemischen Parametern von pharmazeutischen Objekten wie z.B. Tabletten verwendbar sein soll und das erfindungsgemäße Verfahren z.B. als Teil eines Verfahrens zur Prüfung von physikalischen oder chemischen Parametern von pharmazeutischen Objekten wie z.B. Tabletten anwendbar sein soll.

Die Aufgabe wird erfindungsgemäß gelöst durch einen selbstentleerenden Behälter, welcher von zylindrischer oder schüsselartiger Form ist, mit einer den Innenraum des Behälters rotationssymmetrisch oder nicht rotationssysmmetrisch peripher umschließenden Seitenwand, welche einen ersten und einen zweiten Anschlag sowie eine erste und eine zweite Öffnung aufweist, sowie einem Boden, welcher mittels eines Antriebs gegenüber der Seitenwand um eine mit der Mittelachse der Seitenwand (20) zusammenfallende Drehachse wahlweise in beide Drehrichtungen in Rotation versetzbar ist, wobei
- innerhalb des Behälters ein Steg angeordnet ist, welcher sowohl gegenüber dem Boden als auch gegenüber der Seitenwand um eine Schwenkachse schwenkbar gelagert ist,
- die Auslenkung der Schwenkung des Stegs gegenüber der Seitenwand in einer Richtung durch den ersten Anschlag und in der entgegengesetzten Richtung durch den zweiten Anschlag begrenzt ist, so dass sich der Steg bei Anliegen am ersten Anschlag in einer ersten Anschlagstellung und bei Anliegen am zweiten Anschlag in einer zweiten Anschlagstellung befindet und der Steg zwischen diesen beiden Anschlagstellungen lediglich um ein bestimmtes Winkelintervall schwenkbar ist,
- der Steg (12,12') mit dem Boden (11) über einen Kraftschluss oder Reibungsschluss gekoppelt ist, wodurch der Steg (12,12') bei Rotation des Bodens (11) in eine Drehrichtung in die erste Anschlagstellung und bei Rotation des Bodens (11) in die andere Drehrichtung in die zweite Anschlagstellung geschwenkt wird, und/oder der Steg (12,12') mittels eines separaten Antriebs von der ersten in die zweite Anschlagstellung und umgekehrt versetzbar ist,
- und der Steg so geformt ist und die Anschläge so angeordnet sind, dass der Steg in der ersten Anschlagstellung die erste Öffnung blockiert und die zweite Öffnung freigibt und in der zweiten Anschlagstellung die erste Öffnung freigibt und die zweite Öffnung blockiert,
wodurch im Behälter vorhandene Objekte oder im Behälter vorhandenes Material wahlweise je nach Drehrichtung des Bodens den Behälter nur durch die erste oder nur durch die zweite Öffnung verlassen können.

Oben ist der Behälter vorzugsweise offen.

Bevorzugt ist der Boden an seiner dem Innenraum zugewandten Seite konisch, kegelmantelförmig oder konvex ausgebildet, so dass im Behälter vorhandene Objekte oder im Behälter vorhandenes Material schwerkraftbedingt durch entweder die erste oder die zweite Öffnung aus dem Behälter austreten.

Die Kegelspitze oder der höchste Punkt des Bodens fällt bevorzugt mit der Drehachse zusammen.

Gemäß einer Ausführungsform fällt die Kegelspitze oder der höchste Punkt des Bodens nicht mit der Drehachse zusammen, wobei die Oberseite des Bodens aber dennoch so geformt ist, dass im Behälter vorhandene Objekte oder im Behälter vorhandenes Material schwerkraftbedingt durch entweder die erste oder die zweite Öffnung aus dem Behälter austreten.

Bevorzugt ist der Boden an seiner dem Innenraum zugewandten Seite konisch, kegelmantelförmig oder konvex ausgebildet, wobei die Kegelspitze oder der höchste Punkt des Bodens mit der Drehachse zusammenfällt, so dass im Behälter vorhandene Objekte oder im Behälter vorhandenes Material schwerkraftbedingt durch entweder die erste oder die zweite Öffnung aus dem Behälter austreten.

Der Boden kann insbesondere in Schräglage angeordnet sein, wobei sich beide Öffnungen im Bereich des tiefsten Punktes des Behälters befinden, so dass im Behälter vorhandene Objekte oder im Behälter vorhandenes Material schwerkraftbedingt durch entweder die erste oder die zweite Öffnung aus dem Behälter austreten.

Der Boden kann insbesondere so in Schräglage angeordnet sein, dass sich beide Öffnungen im Bereich des tiefsten Punktes des Behälters befinden und im Behälter vorhandene Objekte oder im Behälter vorhandenes Material schwerkraftbedingt durch entweder die erste oder die zweite Öffnung aus dem Behälter austreten.

Gemäß einer bevorzugten Ausführungsform liegt der Steg durchgängig auf dem Boden auf.

Bevorzugt sind an dem Steg im Bereich seiner Unterseite ein Besen oder Borsten zum Abstreifen der Objekte oder des Materials angeordnet, welche auf dem Boden aufstehen.

Gemäß einer bevorzugten Ausführungsform ist der Steg gleichzeitig als Barriere zu fungieren imstande, welche im Behälter vorhandene Objekte oder im Behälter vorhandenes Material daran hindert, an einer vollen Rotation des Bodens teilzunehmen.

Gemäß einer bevorzugten Ausführungsform überragt der Steg die Seitenwand nach außen und ragt in wenigstens eine der Öffnungen hinein, wobei eine der ersten Öffnung zugewandte Kante der Seitenwand als erster Anschlag und eine der zweiten Öffnung zugewandte Kante der Seitenwand als zweiter Anschlag fungiert.

Gemäß einer bevorzugten Ausführungsform weist ein erfindungsgemäßer selbstentleerender Behälter eine Anschlagnase auf, welche zugleich als erster und zweiter Anschlag fungiert.

Gemäß einer bevorzugten Ausführungsform weist ein erfindungsgemäßer selbstentleerender Behälter einen Umschalter zur fernbetätigten Umschaltung der Drehrichtung des Bodens auf.

Gemäß einer Ausführungsform der Erfindung fällt die Schwenkachse mit der Drehachse zusammen.

Gemäß einer Ausführungsform ist der Steg mittels eines separaten Antriebs von der ersten in die zweite Anschlagstellung und umgekehrt versetzbar. In diesem Fall ist ein Kraftschluss oder Reibungsschluss zwischen Steg und Boden verzichtbar, da der Steg unabhängig hiervon in die jeweils gewünschte Anschlagstellung versetzbar ist.

Ein erfindungsgemäßer selbstentleerender Behälter kann insbesondere Bestandteil sein einer Vorrichtung zur Bestimmung wenigstens eines physikalischen oder chemischen Parameters von Objekten, insbesondere von pharmazeutischen Objekten, wie Tabletten, Pillen oder Oblongs. Eine derartige Vorrichtung kann einen Abstreifer zum Abstreifen von Material, Objekten oder deren Bruchstücken von einer Prüfeinrichtung oder einer Unterlage aufweisen; ein erfindungsgemäßer selbstentleerender Behälter kann insbesondere zur Aufnahme des durch den Abstreifer abgestreiften Materials oder Objekts oder dessen Überresten dienen.

Die Aufgabe wird ferner gelöst durch ein Verfahren zum Entleeren eines Behälters, welcher von zylindrischer oder schüsselartiger Form ist, mit
- einer den Innenraum des Behälters rotationssymmetrisch oder nicht roationssysmmetrisch peripher umschließenden Seitenwand, welche einen ersten und einen zweiten Anschlag sowie eine erste und eine zweite Öffnung aufweist, sowie
- einem Boden, welcher mittels eines Antriebs gegenüber der Seitenwand um eine mit der Mittelachse der Seitenwand zusammenfallende Drehachse wahlweise in beide Drehrichtungen in Rotation versetzbar ist,
   unter Verwendung eines Stegs, welcher innerhalb des Behälters angeordnet und sowohl gegenüber dem Boden als auch gegenüber der Seitenwand um eine Schwenkachse schwenkbar gelagert ist, wobei
- die Auslenkung der Schwenkung des Stegs gegenüber der Seitenwand in einer Richtung durch den ersten Anschlag und in der entgegengesetzten Richtung durch den zweiten Anschlag begrenzt ist, so dass sich der Steg bei Anliegen am ersten Anschlag in einer ersten Anschlagstellung und bei Anliegen am zweiten Anschlag in einer zweiten Anschlagstellung befindet und der Steg zwischen diesen beiden Anschlagstellungen lediglich um ein bestimmtes Winkelintervall schwenkbar ist,
- der Steg mit dem Boden über einen Kraftschluss oder Reibungsschluss gekoppelt ist, wodurch der Steg bei Rotation des Bodens in eine Drehrichtung in die erste Anschlagstellung und bei Rotation des Bodens in die andere Drehrichtung in die zweite Anschlagstellung geschwenkt wird, und/oder der Steg mittels eines separaten Antriebs abwechselnd von der ersten in die zweite Anschlagstellung und umgekehrt versetzt wird,
- und der Steg so geformt ist und die Anschläge so angeordnet sind, dass der Steg in der ersten Anschlagstellung die erste Öffnung blockiert und die zweite Öffnung freigibt und in der zweiten Anschlagstellung die erste Öffnung freigibt und die zweite Öffnung blockiert,
wobei die Drehrichtung des Bodens um die Drehachse
- entweder so gewählt wird, dass im Behälter vorhandene Objekte oder im Behälter vorhandenes Material wahlweise je nach Drehrichtung des Bodens den Behälter nur durch die erste Öffnung verlassen können,
- oder so gewählt wird, dass im Behälter vorhandene Objekte oder vorhandenes Material wahlweise je nach Drehrichtung des Bodens den Behälter nur durch die zweite Öffnung verlassen können.

Bevorzugt wird als Boden ein solcher verwendet, welcher konisch, kegelmantelförmig oder konvex ausgebildet ist, so dass im Behälter vorhandene Objekte oder im Behälter vorhandenes Material schwerkraftbedingt durch entweder die erste oder die zweite Öffnung aus dem Behälter austreten.

Insbesondere kann als Boden ein solcher verwendet werden, dessen Kegelspitze oder der höchste Punkt des Bodens mit der Drehachse zusammenfällt.

Gemäß einer Variante wird als Boden ein solcher verwendet, dessen Kegelspitze oder höchster Punkt nicht mit der Drehachse zusammenfällt, wobei die Oberseite des Bodens aber dennoch so geformt ist, dass im Behälter vorhandene Objekte oder im Behälter vorhandenes Material schwerkraftbedingt durch entweder die erste oder die zweite Öffnung aus dem Behälter austreten.

Bevorzugt wird als Boden ein solcher verwendet, welcher konisch, kegelmantelförmig oder konvex ausgebildet ist, wobei die Kegelspitze oder der höchste Punkt des Bodens mit der Drehachse zusammenfällt, so dass im Behälter vorhandene Objekte oder im Behälter vorhandenes Material schwerkraftbedingt durch entweder die erste oder die zweite Öffnung aus dem Behälter austreten.

Der Boden kann vorteilhaft in Schräglage angeordnet werden, wobei sich beide Öffnungen im Bereich des tiefsten Punktes des Behälters befinden, so dass im Behälter vorhandene Objekte oder im Behälter vorhandenes Material schwerkraftbedingt durch entweder die erste oder die zweite Öffnung aus dem Behälter austreten.

Der Boden kann insbesondere so in Schräglage angeordnet werden, dass sich beide Öffnungen im Bereich des tiefsten Punktes des Behälters befinden und im Behälter vorhandene Objekte oder im Behälter vorhandenes Material schwerkraftbedingt durch entweder die erste oder die zweite Öffnung aus dem Behälter austreten.

Als Steg kann vorteilhaft ein solcher verwendet werden, welcher durchgängig auf dem Boden aufliegt.

Gemäß einer Variante wird als Steg kann ein solcher verwendet, an welchem im Bereich seiner Unterseite ein Besen oder Borsten zum Abstreifen der Objekte oder des Materials angeordnet sind, welche auf dem Boden aufstehen.

Der Steg wird gemäß einer Variante gleichzeitig als Barriere verwendet, welche im Behälter vorhandene Objekte oder im Behälter vorhandenes Material daran hindert, an einer vollen Rotation des Bodens teilzunehmen.

Als Steg kann ein solcher verwendet werden, welcher die Seitenwand nach außen überragt und in wenigstens eine der Öffnungen hineinragt, wobei eine der ersten Öffnung zugewandte Kante der Seitenwand als erster Anschlag und eine der zweiten Öffnung zugewandte Kante der Seitenwand als zweiter Anschlag fungiert.

Gemäß einer Variante wird eine Anschlagnase verwendet , welche zugleich als erster und zweiter Anschlag fungiert.

Gemäß einer Variante wird die Drehrichtung des Bodens mittels eines Umschalters umgeschaltet.

Gemäß einer Variante des erfindungsgemäßen Verfahrens fällt die Schwenkachse mit der Drehachse zusammen.

Gemäß einer Variante wird der Steg mittels eines separaten Antriebs abwechselnd von der ersten in die zweite Anschlagstellung und umgekehrt versetzt. Vorzugsweise werden diese Versetzungen des Steges mit der Umschaltung der Rotationsrichtung des Bodens gekoppelt, so dass der separate Antrieb den Kraftschluss oder Reibungsschluss zwischen Steg und Boden ersetzt.

Das erfindungsgemäße Verfahren kann insbesondere Teil eines Verfahrens zur Bestimmung wenigstens eines physikalischen oder chemischen Parameters von Objekten sein, insbesondere von pharmazeutischen Objekten, wie Tabletten, Pillen oder Oblongs.

Kurzbeschreibung der Zeichnung, in welcher schematisch und beispielhaft anhand von Ausführungsbeispielen der Erfindung zeigen:
- Figur 1: einen erfindungsgemäßen selbstentleerenden Behälter mit einem rotierbaren Boden, einer Seitenwand und einem im Inneren des Behälters angeordneten Steg, welcher um die Rotationsachse des Bodens um einen Winkel von mehr als 180° schwenkbar ist,
- Figur 2: einen erfindungsgemäßen selbstentleerenden Behälter, welcher sich von demjenigen von Figur 1 dadurch unterscheidet, dass der Steg um die Rotationsachse des Bodens um einen Winkel von weniger als 180° schwenkbar ist,
- Figur 3: einen erfindungsgemäßen selbstentleerenden Behälter, welcher sich von demjenigen von Figur 2 dadurch unterscheidet, dass der Steg um die Rotationsachse des Bodens nur um einen Winkel von ca. 30° schwenkbar ist,
- Figur 4: einen erfindungsgemäßen selbstentleerenden Behälter, welcher sich von demjenigen von der Figuren 1 bis 3 dadurch unterscheidet, dass der Steg mehr als 50% des Bodens überdeckt, und
- Figur 5: einen erfindungsgemäßen selbstentleerenden Behälter, welcher sich von demjenigen von Figur 3 dadurch unterscheidet, dass der Steg die Seitenwand nach außen überragt.

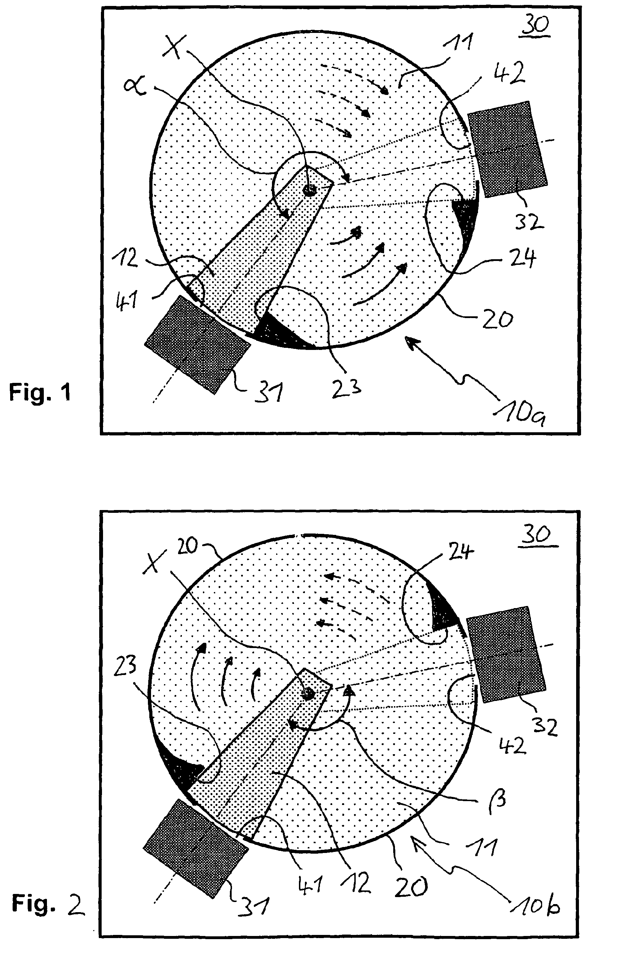

Die Figuren 1 bis 5 sind schematische Darstellungen und beziehen sich auf jeweils eine in Draufsicht von oben dargestellte beispielhafte Ausführungsform eines erfindungsgemäßen selbstentleerenden Behälters. Die Objekte oder das Material, von denen der Behälter sich entleeren soll, sind in den Figuren nicht dargestellt.

Fig. 1 zeigt eine erste Ausführungsform 10a eines erfindungsgemäßen selbstentleerenden Behälters. Dieser ist von im wesentlichen zylindrischer oder schüsselartiger Form und besitzt vorzugsweise eine den Innenraum des Behälters 10a rotationssymmetrisch peripher umschließende Seitenwand 20, welche einen ersten und einen zweiten Anschlag 23,24 sowie eine erste und eine zweite Öffnung 41,42 aufweist. Jeder Anschlag 23,24 ist im Bereich des Randes einer der Öffnungen 41,42 vorzugsweise starr an der Seitenwand 20 angeordnet. Bevorzugt erstreckt sich jede der Öffnungen in der Seitenwand 20 nach unten bis auf das Niveau des Bodens 11.

Der Behälter 10a besitzt ferner einen Boden 11, welcher mittels eines nicht gezeigten, vorzugsweise unter dem Boden 11 befindlichen Antriebs gegenüber der Seitenwand 20 um eine mit der Mittelachse der Seitenwand 20 zusammenfallende Drehachse X wahlweise in beide Drehrichtungen in Rotation versetzbar ist. Die Seitenwand 20 nimmt an dieser Rotation nicht teil. Die eine Drehrichtung (in Fig. 1 gegen den Uhrzeigersinn) des Bodens 11 ist durch drei parallele gekrümmte durchgezogene Pfeile dargestellt; die andere Drehrichtung (in Fig. 1 mit dem Uhrzeigersinn) ist durch drei parallele gekrümmte gestrichelte Pfeile dargestellt.

Innerhalb des Behälters 10a ist auf oder über dem Boden 11 ein Steg 12 angeordnet, welcher um die Drehachse X sowohl gegenüber dem Boden 11 als auch gegenüber der Seitenwand 20 schwenkbar gelagert ist. Die Auslenkung der Schwenkung des Stegs 12 ist hierbei gegenüber der Seitenwand 20 in einer Richtung, nämlich gegen den Uhrzeigersinn, durch den ersten Anschlag 23, und in der entgegengesetzten Richtung durch den zweiten Anschlag 24 begrenzt. Daher befindet sich der Steg 12 bei Anliegen am ersten Anschlag 23 in einer ersten Anschlagstellung. In Fig. 1 ist der Steg 12 in dieser Stellung durchgezogen eingezeichnet. Bei Anliegen am zweiten Anschlag 24 befindet sich der Steg 12 in einer zweiten Anschlagstellung. In Fig. 1 ist der Steg 12 in dieser Stellung punktiert und durchsichtig eingezeichnet. Zwischen diesen beiden Anschlagstellungen ist der Steg 12 lediglich um ein bestimmtes Winkelintervall α schwenkbar, welches im Beispiel von Fig. 1 größer ist als 180°.

Der Steg 12 ist mit dem rotierenden Boden 11 über einen Reibungsschluss gekoppelt, wodurch der Steg 12 an der Rotation des Bodens 11 teilzunehmen versucht. Der Reibungsschluss kann z.B. dadurch realisiert sein, dass der Steg 12 durchgängig auf dem Boden 11 aufliegt, so dass der Boden 11 bei seiner Rotation auf die Unterseite des Stegs 11 eine Reibungskraft ausübt.

Eine andere Möglichkeit zur Herstellung eines Reibungsschlusses besteht darin, dass die Drehachse X durch einen mit dem Boden 11 starr mitrotierenden Bolzen gebildet ist und der Steg 12 mittels einer Rutschkupplung an der Drehachse X angeordnet ist. Eine weitere Möglichkeit zur Herstellung eines Reibungsschlusses besteht darin, dass an dem Steg 12 im Bereich seiner Unterseite ein Besen oder Borsten zum Abstreifen der Objekte oder des Materials angeordnet sind, welche auf dem Boden 11 aufstehen und daher von diesem mit einer Reibungskraft beaufschlagt werden. An die Stelle des Reibungsschlusses kann auch ein nicht reibungsbedingter Kraftschluss treten, z.B. ein solcher, welcher auf Kraftübertragung mittels Magneten beruht.

Auf Grund des Reibungsschlusses wird der Steg 12 durch Rotation des Bodens 11 entgegen dem Uhrzeigersinn in die erste Anschlagstellung und durch Rotation des Bodens 11 in die andere Drehrichtung in die zweite Anschlagstellung geschwenkt.

Erfindungsgemäß ist der Steg 12 so geformt und sind die Anschläge 23,24 so angeordnet, dass der Steg 12
- in der ersten Anschlagstellung die erste Öffnung 41 blockiert (oder zumindest im wesentlichen obstruiert) und die zweite Öffnung 42 freigibt, so dass im Behälter befindliche Objekte oder Material diesen nur durch die zweite Öffnung 42 verlassen können,
- und in der zweiten Anschlagstellung umgekehrt die erste Öffnung 41 freigibt und die zweite Öffnung 42 blockiert (oder zumindest im wesentlichen obstruiert), so dass so dass im Behälter befindliche Objekte oder Material diesen nur durch die erste Öffnung 41 verlassen können.

Der Ausgang aus dem Behälter, welchen die Objekte oder das Material nehmen müssen, ist somit erfindungsgemäß durch Wahl der Rotationsrichtung des Bodens 11 vorgebbar.

Im Beispiel von Fig. 1 ist der Behälter 10a auf einer Montageplatte 30 angeordnet, welche im Bereich jeder der Öffnungen 41,42 je ein Loch 31,32 aufweist, so dass Objekte oder Material, welche den Behälter 10a durch eine der Öffnungen 41,42 verlassen, durch diese Löcher 31 bzw. 32 hindurch z.B. in unter der Montageplatte 30 aufgestellte Auffanggefäße gelangen können.

Um einen Stau der Objekte am Steg 12 zu vermeiden, wird der selbstentleerende Behälter 10a bevorzugt so angeordnet, dass die Objekte bei Einbringen in den selbstentleerenden Behälter 10a nicht auf den vom Winkelintervall α überstrichenen bzw. überstreichbaren Teil des Bodens 11, sondern nur auf den nicht vom Steg 12 überstreichbaren Bereich des Bodens 11 gelangen, also z.B. so in den Behälter 10a eingeworfen werden, dass sie nur im letztgenannten Bereich auf den Boden 11 auftreffen. Bevorzugt ist ein erfindungsgemäßer selbstentleerender Behälter daher so eingerichtet, dass das vom Steg 12 überstreichbare Winkelintervall möglichst klein ist.

Fig. 2 zeigt eine zweite Ausführungsform 10b eines erfindungsgemäßen selbstentleerenden Behälters, bei welchem das vom Steg 12 überstreichbare Winkelintervall gegenüber Fig. 1 kleiner ist. Der Behälter 10b von Fig. 2 unterscheidet sich von dem Behälter 10a von Fig. 10 nur durch eine andere Anordnung der Anschläge 23 und 24, die nun so gewählt ist, dass der Steg 12 durch Rotation des Bodens 11 im Uhrzeigersinn (in Fig. 2 durch durchgezogene gekrümmte Pfeile dargestellt) in die erste Anschlagstellung und durch Rotation des Bodens 11 in die andere Drehrichtung (in Fig. 2 durch gestrichelte gekrümmte Pfeile dargestellt) in die zweite Anschlagstellung geschwenkt wird. Zwischen diesen beiden Anschlagstellungen ist der Steg 12 lediglich um ein bestimmtes Winkelintervall β schwenkbar, welches kleiner ist als das Winkelintervall α von Fig. 1.

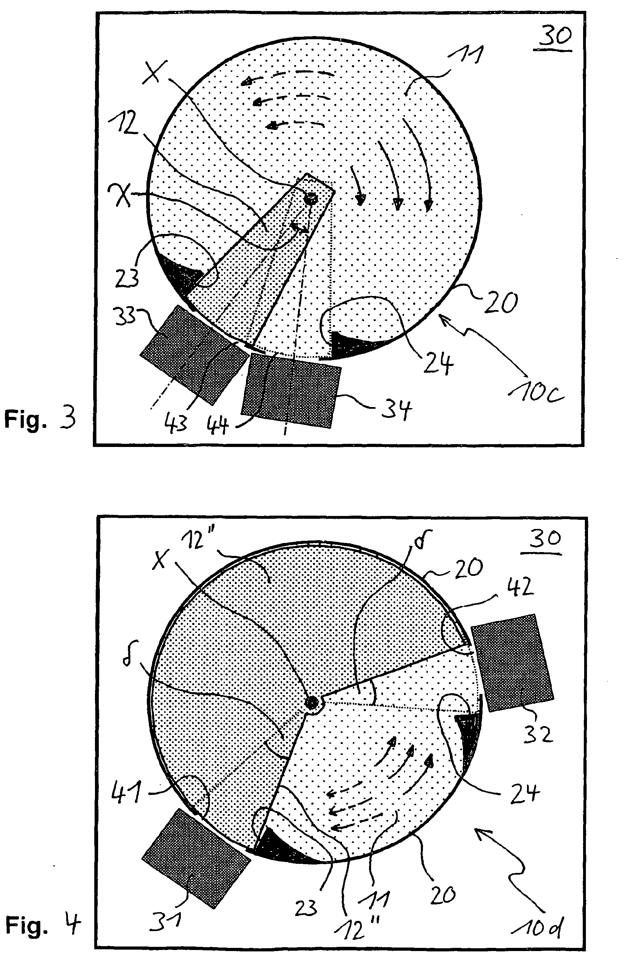

Fig. 3 zeigt eine dritte Ausführungsform 10c eines erfindungsgemäßen selbstentleerenden Behälters mit einem nochmals verkleinerten vom Steg 12 überstreichbaren Winkelintervall. Der Behälter 10c unterscheidet sich von dem Behälter 10a von Fig. 1 nur dadurch, dass anstelle der Öffnungen 41 und 42 in Fig. 3 an anderen Stellen Öffnungen 43 und 44 in der Seitenwand 20 vorhanden sind und dass die Anschläge 23 und 24 wiederum anders angeordnet sind, deren Anordnung hier wiederum so gewählt ist, dass der Steg 12 durch Rotation des Bodens 11 im Uhrzeigersinn (in Fig. 3 durch durchgezogene gekrümmte Pfeile dargestellt) in die erste Anschlagstellung und durch Rotation des Bodens 11 in die andere Drehrichtung (in Fig. 3 durch gestrichelte gekrümmte Pfeile dargestellt) in die zweite Anschlagstellung geschwenkt wird. Zwischen diesen beiden Anschlagstellungen ist der Steg 12 lediglich um ein bestimmtes Winkelintervall χ schwenkbar, welches in vorliegenden Beispiel ca. 30° beträgt. Anstelle der Löcher 31 und 32 sind in Fig. 3 an anderen Stellen Löcher 33 und 33 in der Montageplatte 30 vorhanden.

Fig. 4 zeigt eine vierte Ausführungsform 10d eines erfindungsgemäßen selbstentleerenden Behälters. Dieser unterscheidet sich von dem Behälter 10a von Fig. 1 dadurch, dass anstelle des Stegs 12 ein wesentlich größerer Steg 12" vorhanden ist, welcher so geformt ist dass er in der ersten Anschlagstellung die erste Öffnung 41 blockiert und bis in den Bereich des Randes der zweiten Öffnung 42 reicht, diese aber nicht blockiert. Ein Vorteil gegenüber den Behälter 10a von Fig. 1 besteht darin, dass die Übergangszeit von der ersten zur zweiten Anschlagstellung bzw. umgekehrt verringert ist, da hierzu ein Schwenkung des Stegs 12" nur um einen im Vergleich zum Winkelintervall α wesentlich kleineren Winkel nötig ist.

Die Anschläge 23, 24 der Figuren 10-13 ragen in das Innere der Behälter 10a, 10b, 10c, 10d hinein.

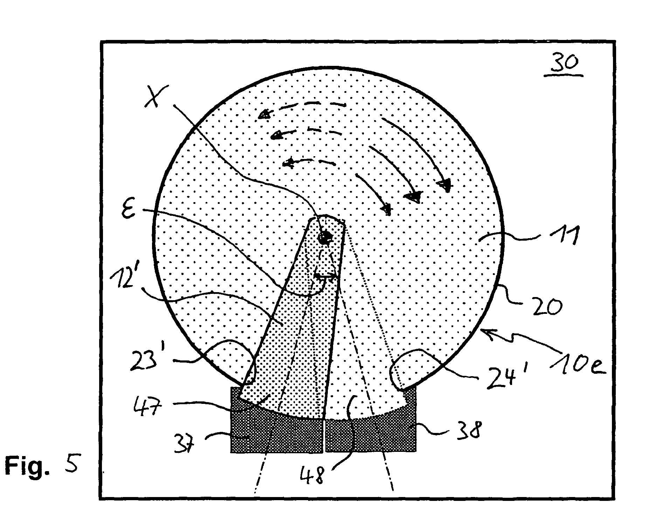

Fig. 5 zeigt eine fünfte Ausführungsform 10e eines erfindungsgemäßen selbstentleerenden Behälters, bei welchem keine Anschläge in das Innere des Behälters hineinreichen. Der Behälter 10e von Fig. 5 unterscheidet sich von dem Behälter 10a von Fig. 1 dadurch, dass anstelle der Öffnungen 41 und 42 in Fig. 5 an anderen Stellen Öffnungen 47 und 48 in der Seitenwand 20 vorhanden sind, welche ineinander übergehen und somit verschiedene Enden ein- und derselben großen Öffnung 47,48 darstellen. Anstelle des Stegs 12 ist ein etwas längerer Steg 12' vorhanden, welcher die Seitenwand 20 nach außen überragt und in zumindest eine der beiden Öffnungen 47, 48 hineinragt. Hierdurch fungiert die der ersten Öffnung 47 zugewandte Kante der Seitenwand 20 als erster Anschlag 23' und die der zweiten Öffnung 48 zugewandte Kante der Seitenwand 20 als zweiter Anschlag 24'. Zwischen den beiden hierdurch festgelegten Anschlagstellungen (von welche ebenfalls die eine durchgezogen und die andere gepunktet eingezeichnet ist) ist der Steg 12' um ein bestimmtes Winkelintervall ε schwenkbar, welches gegenüber dem Winkelintervall α von Fig. 1 vorteilhafterweise klein ist.

Bevorzugt erstreckt sich jede der Öffnungen 41-48 in der Seitenwand 20 nach unten bis auf das Niveau des Bodens 11.

Die Stege 12, 12', 12" sind bevorzugt gleichzeitig als Barriere zu fungieren imstande, welche im Behälter vorhandene Objekte oder im Behälter vorhandenes Material daran hindert, an einer vollen Rotation des Bodens 11 teilzunehmen. Die Stege können hierzu bevorzugt in Richtung parallel zur Drehachse X eine solche Ausdehnung (Dicke) aufweisen, welche großer ist als der Durchmesser oder die Horizontalausdehnung der Objekte. An den Stegen 12, 12' ,12" können im Bereich ihrer Unterseite je ein Besen oder Borsten zum Abstreifen der Objekte oder des Materials angeordnet sein, welche auf dem Boden 11 aufstehen und diesen somit fortlaufend reinigen.

Die Stege 12,12',12" können ferner eine geneigte, konvexe oder dachartig geformte Oberseite aufweisen, von welcher Objekte, die auf den Steg anstatt in auf den Boden 11 gefallen sind, seitlich auf den Boden 11 ableiten.

Der Boden 11 ist an seiner dem Innenraum des Behälters zugewandten Seite bevorzugt konisch, kegelmantelförmig, konvex oder dachartig ausgebildet, wobei die Kegelspitze oder der höchste Punkt des Bodens 11 mit der Drehachse X zusammenfällt, so dass im Behälter vorhandene Objekte oder im Behälter vorhandenes Material schwerkraftbedingt am Rand des Bodens 11 sammeln und durch entweder die erste oder die zweite Öffnung aus dem Behälter austreten.

Gemäß einer weiteren Ausführungsform ist der Boden 11 in Schräglage angeordnet, so dass sich im Behälter vorhandene Objekte oder im Behälter vorhandenes Material schwerkraftbedingt im Bereich des tiefsten Punktes des Behälters sammeln, wobei sich beide Öffnungen ebenfalls im Bereich des tiefsten Punktes des Behälters befinden, so dass die Objekte oder das Material schwerkraftbedingt je nach Rotationssinn des Bodens durch entweder die erste oder die zweite Öffnung aus dem Behälter austreten.

Der erfindungsgemäße Behälter kann sehr vorteilhaft dazu eingesetzt werden, verschiedene Arten von Objekten oder Materialien zu trennen, insbesondere in Verbindung mit einer erfindungsgemäßen Vorrichtung zur Bestimmung wenigstens eines physikalischen oder chemischen Parameters von Objekten, insbesondere von pharmazeutischen Objekten, wie Tabletten, Pillen oder Oblongs.

In einer solchen Vorrichtung kann der erfindungsgemäße selbstentleerende Behälter insbesondere die Funktion eines Zwischenbehälters übernehmen. Beispielsweise können hierbei mittels seiner derartigen Vorrichtung zunächst 1000 für die Umwelt unbedenkliche Tabletten geprüft werden, wobei alle diese Tabletten oder deren Überreste schließlich im Zwischenbehälter, welcher durch einen erfindungsgemäßen selbstentleerenden Behälter gebildet ist, landen. Während der Prüfung dieser 1000 ungiftigen Tabletten wird z.B. die Rotationsrichtung des Bodens 11 im Uhrzeigersinn gewählt, so dass alle 1000 Tabletten oder deren Überreste den Behälter durch die erste Öffnung verlassen und von dort in ein Auffanggefäß gelangen.

Anschließend sollen z.B. 500 für die Umwelt bedenkliche Objekte wie etwa Mottenkugeln oder Hormonpräparate mit der Vorrichtung geprüft werden. Hierfür wird nun die Rotationsrichtung des Bodens 11 in die entgegengesetzte Richtung umgeschaltet, so dass alle Mottenkugeln oder deren Überreste den Behälter durch die zweite Öffnung verlassen und von dort in ein luftdicht verschließbares Entsorgungsgefäß gelangen und ohne weitere Maßnahmen zur Trennung von giftigem und ungiftigem Abfall einer fachgerechten Entsorgung zugeführt werden können. Vorteilhafterweise braucht die Vorrichtung also beim Übergang von der Prüfung der Tabletten zur Prüfung der Mottenkugeln weder angehalten noch zur Entnahme oder Entleerung von Behältern geöffnet zu werden.

### Gewerbliche Anwendbarkeit:

Die Erfindung ist gewerblich anwendbar z.B. im Bereich der Pharmatechnik oder des Recyclings.

### Liste der Bezugszeichen:

- X: Drehachse von 11 und Schwenkachse von 12
- 10a-10e: Behälter
- 11: Boden
- 12,12',12": Stege
- 20: Seitenwand
- 23,23': erste Anschläge
- 24,24': zweite Anschläge
- 30: Montageplatte
- 31-38: Löcher in 30
- 41,43,45,47: erste Öffnung in 20
- 42,44,46,48: zweite Öffnung in 20
- α,β,χ,δ,ε: Winkelintervalle für die Schwenkung von 12

## Patentansprüche

1. Selbstentleerender Behälter (10a,10b, 10c,10d, 10e), welcher von zylindrischer oder schüsselartiger Form ist, mit
- einer den Innenraum des Behälters (10a,10b,10c,10d,10e) rotationssymmetrisch oder nicht rotationssysmmetrisch peripher umschließenden Seitenwand (20), welche einen ersten und einen zweiten Anschlag (23,24, 23',24', 25) sowie eine erste und eine zweite Öffnung (41,43,45,47, 42,44,46,48) aufweist, sowie
- einem Boden (11), welcher mittels eines Antriebs gegenüber der Seitenwand (20) um eine mit der Mittelachse der Seitenwand (20) zusammenfallende Drehachse (X) wahlweise in beide Drehrichtungen in Rotation versetzbar ist,
wobei
- innerhalb des Behälters (10a,10b,10c,10d,10e) ein Steg (12,12') angeordnet ist, welcher sowohl gegenüber dem Boden (11) als auch gegenüber der Seitenwand (20) um eine Schwenkachse schwenkbar gelagert ist,
- die Auslenkung der Schwenkung des Stegs (12,12') gegenüber der Seitenwand (20) in einer Richtung durch den ersten Anschlag (23,23',25) und in der entgegengesetzten Richtung durch den zweiten Anschlag (24,24',25) begrenzt ist, so dass sich der Steg (12,12') bei Anliegen am ersten Anschlag (23,23',25) in einer ersten Anschlagstellung und bei Anliegen am zweiten Anschlag (24,24',25) in einer zweiten Anschlagstellung befindet und der Steg (12,12') zwischen diesen beiden Anschlagstellungen lediglich um ein bestimmtes Winkelintervall (α,β,χ,δ,ε) schwenkbar ist,
- der Steg (12,12') mit dem Boden (11) über einen Kraftschluss oder Reibungsschluss gekoppelt ist, wodurch der Steg (12,12') bei Rotation des Bodens (11) in eine Drehrichtung in die erste Anschlagstellung und bei Rotation des Bodens (11) in die andere Drehrichtung in die zweite Anschlagstellung geschwenkt wird, und/oder der Steg (12,12') mittels eines separaten Antriebs von der ersten in die zweite Anschlagstellung und umgekehrt versetzbar ist,
- und der Steg (12,12') so geformt ist und die Anschläge (23,24, 23',24', 25) so angeordnet sind, dass der Steg (12,12') in der ersten Anschlagstellung die erste Öffnung (41,43,45,47) blockiert und die zweite Öffnung (42,44,46,48) freigibt und in der zweiten Anschlagstellung die erste Öffnung (41,43,45,47) freigibt und die zweite Öffnung (42,44,46,48) blockiert,
wodurch im Behälter (10a,10b,10c,10d,10e) vorhandene Objekte oder im Behälter (10a,10b,10c,10d,10e) vorhandenes Material wahlweise je nach Drehrichtung des Bodens (11) den Behälter (10,10b,10c,10d,10e) nur durch die erste oder nur durch die zweite Öffnung (41,43,45,47, 42,44,46,48) verlassen können.

2. Selbstentleerender Behälter nach Anspruch 1, **dadurch gekennzeichnet, dass** der Boden (11) an seiner dem Innenraum zugewandten Seite konisch, kegelmantelförmig oder konvex ausgebildet ist, so dass im Behälter (10a, 10b, 10c, 10d, 10e) vorhandene Objekte oder im Behälter (10a, 10b, 10c, 10d,10e) vorhandenes Material schwerkraftbedingt durch entweder die erste oder die zweite Öffnung (41,43,45,47, 42,44,46,48) aus dem Behälter (10) austreten.

3. Selbstentleerender Behälter nach Anspruch 2, **dadurch gekennzeichnet, dass** die Kegelspitze oder der höchste Punkt des Bodens mit der Drehachse (X) zusammenfällt.

4. Selbstentleerender Behälter nach einem der vorigen Ansprüche, **dadurch gekennzeichnet, dass** der Boden (11) so in Schräglage angeordnet ist, dass sich beide Öffnungen (41,43,45,47, 42,44,46,48) im Bereich des tiefsten Punktes des Behälters (10a,10b,10c,10d,10e) befinden und im Behälter (10) vorhandene Objekte oder im Behälter (10a,10b, 10c,10d, 10e) vorhandenes Material schwerkraftbedingt durch entweder die erste oder die zweite Öffnung (41,43,45,47, 42,44,46,48) aus dem Behälter (10a,10b,10c,10d,10e) austreten.

5. Selbstentleerender Behälter nach einem der vorigen Ansprüche, **dadurch gekennzeichnet, dass** der Steg (12,12') durchgängig auf dem Boden (11) aufliegt.

6. Selbstentleerender Behälter nach einem der vorigen Ansprüche, **dadurch gekennzeichnet, dass** an dem Steg (12,12') im Bereich seiner Unterseite ein Besen oder Borsten zum Abstreifen der Objekte oder des Materials angeordnet sind, welche auf dem Boden (11) aufstehen.

7. Selbstentleerender Behälter nach einem der vorigen Ansprüche, **dadurch gekennzeichnet, dass** der Steg (12,12') gleichzeitig als Barriere (12,12') zu fungieren imstande ist, welche im Behälter (10a,10b, 10c, 10d, 10e) vorhandene Objekte oder im Behälter (10a,10b,10c,10d,10e) vorhandenes Material daran hindert, an einer vollen Rotation des Bodens (11) teilzunehmen.

8. Selbstentleerender Behälter nach einem der vorigen Ansprüche, **dadurch gekennzeichnet, dass** der Steg (12,12') die Seitenwand (20) nach außen überragt und in wenigstens eine der Öffnungen (47,48) hineinragt, wobei eine der ersten Öffnung (47) zugewandte Kante der Seitenwand (20) als erster Anschlag (23') und eine der zweiten Öffnung (48) zugewandte Kante der Seitenwand (20) als zweiter Anschlag (24') fungiert.

9. Selbstentleerender Behälter nach einem der vorigen Ansprüche, **gekennzeichnet durch** eine Anschlagnase (25), welche zugleich als erster und zweiter Anschlag fungiert.

10. Selbstentleerender Behälter nach einem der vorigen Ansprüche, **gekennzeichnet durch** einen Umschalter zur fernbetätigten Umschaltung der Drehrichtung des Bodens (11).

11. Selbstentleerender Behälter nach einem der vorigen Ansprüche, **dadurch gekennzeichnet, dass** die Schwenkachse mit der Drehachse (X) zusammenfällt.

12. Verfahren zum Entleeren eines Behälters (10a,10b,10c,10d,10e), welcher von zylindrischer oder schüsselartiger Form ist, mit
- einer den Innenraum des Behälters (10a,10b,10c,10d,10e) rotationssymmetrisch oder nicht roationssysmmetrisch peripher umschließenden Seitenwand (20), welche einen ersten und einen zweiten Anschlag (23,24, 23',24', 25) sowie eine erste und eine zweite Öffnung (41,42,45,47, 42,44,46,48) aufweist, sowie
- einem Boden (11), welcher mittels eines Antriebs gegenüber der Seitenwand (20) um eine mit der Mittelachse der Seitenwand (20) zusammenfallende Drehachse (X) wahlweise in beide Drehrichtungen in Rotation versetzbar ist,
unter Verwendung eines Stegs (12,12'), welcher innerhalb des Behälters (10a,1 0b,10c,10d,10e) angeordnet und sowohl gegenüber dem Boden (11) als auch gegenüber der Seitenwand (20) um eine Schwenkachse schwenkbar gelagert ist, wobei
- die Auslenkung der Schwenkung des Stegs (12,12') gegenüber der Seitenwand (20) in einer Richtung durch den ersten Anschlag (23,23',25) und in der entgegengesetzten Richtung durch den zweiten Anschlag (24,24',25) begrenzt ist, so dass sich der Steg (12,12') bei Anliegen am ersten Anschlag (23,23',25) in einer ersten Anschlagstellung und bei Anliegen am zweiten Anschlag (24,24',25) in einer zweiten Anschlagstellung befindet und der Steg (12,12') zwischen diesen beiden Anschlagstellungen lediglich um ein bestimmtes Winkelintervall (α,β,χ,δ,ε) schwenkbar ist,
- der Steg (12,12') mit dem Boden (11) über einen Kraftschluss oder Reibungsschluss gekoppelt ist, wodurch der Steg (12,12') bei Rotation des Bodens (11) in eine Drehrichtung in die erste Anschlagstellung und bei Rotation des Bodens (11) in die andere Drehrichtung in die zweite Anschlagstellung geschwenkt wird, und/oder der Steg (12,12') mittels eines separaten Antriebs abwechselnd von der ersten in die zweite Anschlagstellung und umgekehrt versetzt wird,
- und der Steg (12,12') so geformt ist und die Anschläge (23,24, 23',24', 25) so angeordnet sind, dass der Steg (12,12') in der ersten Anschlagstellung die erste Öffnung (41,43,45,47) blockiert und die zweite Öffnung (42,44,46,48) freigibt und in der zweiten Anschlagstellung die erste l5ffnung (41,43,45,47) freigibt und die zweite Öffnung (42,44,46,48) blockiert,
wobei die Drehrichtung des Bodens (11) um die Drehachse (X)
- entweder so gewählt wird, dass im Behälter (10a,10b,10c,10d,10e) vorhandene Objekte oder im Behälter (10a,10b,10c,10d,10e) vorhandenes Material wahlweise je nach Drehrichtung des Bodens (11) den Behälter (10,10b, 10c,10d,10e) nur durch die erste Öffnung (41,43,45,47) verlassen können,
- oder so gewählt wird, dass im Behälter (10a,10b,10c,10d,10e) vorhandene Objekte oder vorhandenes Material wahlweise je nach Drehrichtung des Bodens (11) den Behälter (10,10b, 10c,10d,10e) nur durch die zweite Öffnung (42,44,46,48) verlassen können.

13. Verfahren nach Anspruch 12 , **dadurch gekennzeichnet,**
**dass** als Boden (11) ein solcher verwendet wird, welcher konisch, kegelmantelförmig oder konvex ausgebildet ist, so dass im Behälter (10a,10b,10c,10d,10e) vorhandene Objekte oder im Behälter (10a,10b,10c,10d,10e) vorhandenes Material schwerkraftbedingt durch entweder die erste oder die zweite Öffnung (41,43,45,47, 42,44,46,48) aus dem Behälter (10) austreten.

14. Verfahren nach Anspruch 13, **dadurch gekennzeichnet,**
**dass** dass als Boden ein solcher verwendet wird, dessen Kegelspitze oder der höchste Punkt des Bodens (11) mit der Drehachse (X) zusammenfällt.

15. Verfahren nach einem der Ansprüche 12 bis 14, **dadurch gekennzeichnet,**
**dass** der Boden (11) so in Schräglage angeordnet wird, dass sich beide Öffnungen (41,43,45,47, 42,44,46,48) im Bereich des tiefsten Punktes des Behälters (10a, 10b,10c,10d, 10e) befinden und im Behälter (10) vorhandene Objekte oder im Behälter (10a,10b, 10c, 10d, 10e) vorhandenes Material schwerkraftbedingt durch entweder die erste oder die zweite Öffnung (41,43,45,47, 42,44,46,48) aus dem Behälter (10a,10b,10c,10d,10e) austreten.

16. Verfahren nach einem der Ansprüche 12 bis 15, **dadurch gekennzeichnet,**
**dass** als Steg (12,12') ein solcher verwendet wird, welcher durchgängig auf dem Boden (11) aufliegt.

17. Verfahren nach Anspruch 12, **dadurch gekennzeichnet,**
**dass** als Steg (12,12') ein solcher verwendet wird, an welchem im Bereich seiner Unterseite ein Besen oder Borsten zum Abstreifen der Objekte oder des Materials angeordnet sind, welche auf dem Boden (11) aufstehen.

18. Verfahren nach einem der Ansprüche 12 bis 17, **dadurch gekennzeichnet,**
**dass** der Steg (12,12') gleichzeitig als Barriere (12,12') verwendet wird, welche im Behälter (10a,10b,10c,10d,10e) vorhandene Objekte oder im Behälter (10a,10b,10c,10d,10e) vorhandenes Material daran hindert, an einer vollen Rotation des Bodens (11) teilzunehmen.

19. Verfahren nach einem der Ansprüche 12 bis 18, **dadurch gekennzeichnet,**
**dass** als Steg (12') ein solcher verwendet wird, welcher die Seitenwand (20) nach außen überragt und in wenigstens eine der Öffnungen (47,48) hineinragt, wobei eine der ersten Öffnung (47) zugewandte Kante der Seitenwand (20) als erster Anschlag (23') und eine der zweiten Öffnung (48) zugewandte Kante der Seitenwand (20) als zweiter Anschlag (24') fungiert.

20. Verfahren nach einem der Ansprüche 12 bis 19, **dadurch gekennzeichnet, dass** eine Anschlagnase (25) verwendet wird, welche zugleich als erster und zweiter Anschlag fungiert.

21. Verfahren nach einem der Ansprüche 12 bis 20, **dadurch gekennzeichnet, dass** die Drehrichtung des Bodens (11) mittels eines Umschalters umgeschaltet wird.

22. Verfahren nach einem der Ansprüche 12 bis 21, **dadurch gekennzeichnet, dass** die Schwenkachse mit der Drehachse (X) zusammenfällt.

## Claims

1. Self-emptying container (10a,10b,10c,10d,10e) that is cylindrical or bowl-shaped, comprising
• a side wall (20) that peripherally surrounds the interior of the container (10a,10b,10c,10d,10e) rotation-symmetrically or non-rotation-symmetrically, said side wall having a first and a second stop (23,24, 23',24', 25) as well as a first and a second opening (41,43,45,47, 42,44,46,48), as well as
• a base (11) that, by means of a drive, can be selectively made to rotate relative to the side wall (20) in both directions around a rotational axis (X) that coincides with the center axis of the side wall (20),
whereby,
• inside the container (10a,10b,10c,10d,10e), there is a vane (12,12') that is pivotally mounted around a pivoting axis relative to the base (11) as well as to the side wall (20),
• the deflection of the vane (12,12') relative to the side wall (20) is restricted in one direction by the first stop (23,23',25) and in the opposite direction by the second stop (24,24',25), so that, when the vane (12,12') comes to rest against the first stop (23,23',25), it is in a first stop position and, when it comes to rest against the second stop (24,24',25), it is in a second stop position, and the vane (12,12') can only be pivoted between these two stop positions by a certain angle interval (α,β,χ,δ,ε),
• the vane (12,12') is coupled positively or non-positively to the base (11), as a result of which, when the base (11) rotates in one rotational direction, the vane (12,12') is pivoted into the first stop position and, when the base (11) rotates in the other rotational direction, it is pivoted into the second stop position, and/or the vane (12,12') can be moved by means of a separate drive from the first stop position into the second stop position and vice versa,
• and the vane (12,12') is shaped in such a way and the stops (23,24, 23',24', 25) are arranged in such a way that, in the first stop position, the vane (12,12') blocks the first opening (41,43,45,47) while releasing the second opening (42,44,46,48) and, in the second stop position, it releases the first opening (41,43,45,47) while blocking the second opening (42,44,46,48), as a result of which objects present in the container (10a, 10b, 10c, 10d,10e) or material present in the container (10a,10b,10c,10d,10e) can leave the container (10a,10b,10c,10d,10e) as desired, either only through the first opening (41,43,45,47) or only through the second opening (42,44,46,48) as a function of the direction of rotation of the base (11).

2. The self-emptying container according to Claim 1, **characterized in that** the base (11) is configured to be conical, like the envelope of a cone or convex on its side facing the interior, so that objects present in the container (10a,10b,10c,10d,10e) or material present in the container (10a,10b,10c,10d,10e) exit the container (10) due to the force of gravity either through the first opening (41,43,45,47) or through the second opening (42,44,46,48).

3. The self-emptying container according to Claim 2, **characterized in that** cone tip or the highest point of the base coincides with the rotational axis (X).

4. The self-emptying container according to any of the preceding claims, **characterized in that** the base (11) is arranged in such an inclined position that both openings (41,43,45,47, 42,44,46,48) are located in the area of the lowest point of the container (10a,10b,10c,10d,10e) and objects present in the container (10) or material present in the container (10a,10b,10c,10d,10e) exit the container (10a,10b,10c,10d,10e) due to the force of gravity either through the first opening (41,43,45,47) or through the second opening (42,44,46,48).

5. The self-emptying container according to any of the preceding claims, **characterized in that** the vane (12,12') rests continuously on the base (11).

6. The self-emptying container according to any of the preceding claims, **characterized in that** a brush or a sweeper is arranged on the vane (12,12') in the area of its bottom for purposes of scraping the objects or material standing upright on the base (11).

7. The self-emptying container according to any of the preceding claims, **characterized in that** the vane (12,12') can concurrently function as a barrier (12,12') that prevents objects present in the container (10a,10b,10c,10d,10e) or material present in the container (10a,10b,10c,10d,10e) from participating in a full rotation of the base (11).

8. The self-emptying container according to any of the preceding claims, **characterized in that** the vane (12,12') projects towards the outside beyond the side wall (20) and protrudes into at least one of the openings (47, 48), whereby an edge of the side wall (20) facing the first opening (47) functions as the first stop (23') and an edge of the side wall (20) facing the second opening (48) functions as the second stop (24').

9. The self-emptying container according to any of the preceding claims, **characterized by** a stop tab (25) that concurrently functions as the first and the second stops.

10. The self-emptying container according to any of the preceding claims, **characterized by** a changeover switch to remotely switch over the rotational direction of the base (11).

11. The self-emptying container according to any of the preceding claims, **characterized in that** the pivoting axis coincides with the rotational axis (X).

12. A method for emptying a container (10a,10b,10c,10d,10e) that is cylindrical or bowl-shaped, comprising
• a side wall (20) that peripherally surrounds the interior of the container (10a,10b,10c,10d,10e) rotation-symmetrically or non-rotation-symmetrically, said side wall having a first and a second stop (23,24, 23',24', 25) as well as a first and a second opening (41,43,45,47, 42,44,46,48), as well as
• a base (11) that, by means of a drive, can be selectively made to rotate relative to the side wall (20) in both directions around a rotational axis (X) that coincides with the center axis of the side wall (20)
entailing the use of a vane (12,12') that is arranged inside the container (10a,10b,10c,10d,10e) and that is pivotally mounted around a pivoting axis relative to the base (11) as well as to the side wall (20), whereby
• the deflection of the vane (12,12') relative to the side wall (20) is restricted in one direction by the first stop (23,23',25) and in the opposite direction by the second stop (24,24',25), so that, when the vane (12,12') comes to rest against the first stop (23,23',25), it is in a first stop position and, when it comes to rest against the second stop (24,24',25), it is in a second stop position, and the vane (12,12') can only be pivoted between these two stop positions by a certain angle interval (α,β,χ,δ,ε),
• the vane (12,12') is coupled positively or non-positively to the base (11), as a result of which, when the base (11) rotates in one rotational direction, the vane (12,12') is pivoted into the first stop position and, when the base (11) rotates in the other rotational direction, it is pivoted into the second stop position, and/or the vane (12,12') is alternatingly moved by means of a separate drive from the first stop position into the second stop position and vice versa,
• and the vane (12,12') is shaped in such a way and the stops (23,24, 23',24', 25) are arranged in such a way that, in the first stop position, the vane (12,12') blocks the first opening (41,43,45,47) while releasing the second opening (42,44,46,48) and, in the second stop position, it releases the first opening (41,43,45,47) while blocking the second opening (42,44,46,48),
whereby the direction of rotation of the base (11) around the rotational axis (X) is selected in such way that either
• objects present in the container (10a,10b,10c,10d,10e) or material present in the container (10a,10b,10c,10d,10e) can be selected to leave the container (10a,10b,10c,10d,10e) only through the first opening (41,43,45,47)
• or else objects present in the container (10a,10b,10c,10d,10e) or material present in the container (10a,10b,10c,10d,10e) can be selected to leave the container (10a,10b,10c,10d,10e) only through the second opening (42,44,46,48).

13. The method according to Claim 12, **characterized in that** the base (11) used is one configured to be conical, like the envelope of a cone or convex on its side facing the interior, so that objects present in the container (10a,10b,10c,10d,10e) or material present in the container (10a,10b,10c,10d,10e) exit the container (10) due to the force of gravity either through the first opening (41,43,45,47) or through the second opening (42,44,46,48).

14. The method according to Claim 13, **characterized in that** the base used is one whose cone tip or the highest point of the base (11) coincides with the rotational axis (X).

15. The method according to any of Claims 12 to 14, **characterized in that** the base (11) is arranged in such an inclined position that both openings (41,43,45,47, 42,44,46,48) are located in the area of the lowest point of the container (1 0a,1 0b, 10c, 10d, 10e) and objects present in the container (10) or material present in the container (10a,10b,10c,10d,10e) exit the container (10a,10b,10c,10d,10e) due to the force of gravity either through the first opening (41,43,45,47) or through the second opening (42,44,46,48).

16. The method according to any of Claims 12 to 15, **characterized in that** the vane (12,12') used is one that rests continuously on the base (11).

17. The method according to Claim 12, **characterized in that** the vane (12,12') used is one that has a brush or a sweeper in the area of its bottom for purposes of scraping the objects or material standing upright on the base (11).

18. The method according to any of Claims 12 to 17, **characterized in that** the vane (12,12') is concurrently used as a barrier (12,12') that prevents objects present in the container (10a,10b,10c,10d,10e) or material present in the container (10a,10b,10c,10d,10e) from participating in a full rotation of the base (11).

19. The method according to any of Claims 12 to 18, **characterized in that** the vane (12,12') used is one that projects towards the outside beyond the side wall (20) and protrudes into at least one of the openings (47, 48), whereby an edge of the side wall (20) facing the first opening (47) functions as the first stop (23') and an edge of the side wall (20) facing the second opening (48) functions as the second stop (24').

20. The method according to any of Claims 12 to 19, **characterized in that** a stop tab (25) is used that concurrently functions as the first and the second stops.

21. The method according to any of Claims 12 to 20, **characterized in that** the rotational direction of the base (11) is switched over by means of a changeover switch.

22. The method according to any of Claims 12 to 21, **characterized in that** the pivoting axis coincides with the rotational axis (X).

## Revendications

1. Contenant à auto-évacuation (10a,10b,10c,10d,10e) sous forme de cylindre ou de bol comprenant
- une paroi latérale (20) entourant périphériquement de façon rotationnellement symétrique ou non l'espace intérieur du contenant (10a,10b,10c,10d,10e) et présentant une première et une deuxième butée (23,24, 23',24', 25) ainsi qu'un premier et un deuxième orifice (41,43,45,47, 42,44,46,48)
- ainsi qu'un fond (11) lequel, au moyen d'un entraînement, peut être mis en rotation par rapport à la paroi latérale (20) dans l'un ou l'autre des deux sens autour d'un axe de rotation (X) qui coïncide avec l'axe central de la paroi latérale (20),
dans lequel
- une entretoise (12,12') disposée à l'intérieur du contenant (10a,10b,10c,10d,10e) est logée de façon pivotante autour d'un axe de pivotement soit par rapport au fond (11) soit par rapport à la paroi latérale (20),
- la déviation du pivotement de l'entretoise (12,12') par rapport à la paroi latérale (20) est limitée dans l'un des sens par la première butée (23,23',25) et dans le sens opposé par la deuxième butée (24,24',25), de sorte que l'entretoise (12,12'), lorsqu'elle s'appuie contre la première butée (23,23',25), se trouve dans une première position de butée et lorsqu'elle s'appuie contre la deuxième butée (24,24',25), se trouve dans une deuxième position de butée, l'entretoise (12,12') ne pouvant pivoter que dans un intervalle d'angle déterminé (α,β,χ,δ,ε) entre ces deux positions de butée,
- l'entretoise (12,12') est couplée au fond (11) par adhérence ou par friction, si bien que, lorsque le fond (11) tourne dans l'un des sens de rotation, l'entretoise (12,12') se déplace dans la première position de butée, et, lorsque le fond (11) tourne dans l'autre sens de rotation, elle se déplace dans la deuxième position de butée, et/ou l'entretoise (12,12') peut être déplacée de la première position de butée vers la deuxième position de butée et vice versa au moyen d'un entraînement séparé,
- et dans lequel l'entretoise (12,12') est conformée et les butées (23,24, 23',24', 25) sont disposées de telle manière que l'entretoise (12,12'), dans la première position de butée, bloque le premier orifice (41,43,45,47) et débloque le deuxième orifice (42,44,46,48), et dans la deuxième position de butée, débloque le premier orifice (41,43,45,47) et bloque le deuxième orifice (42,44,46,48),
ce qui a pour effet que, en fonction du sens de rotation du fond (11), les objets ou matières présents dans le contenant (10a,10b,10c,10d,10e) peuvent sortir du contenant (10,10b,10c,10d,10e) uniquement par le premier ou uniquement par le deuxième orifice (41,43,45,47, 42,44,46,48).

2. Contenant à auto-évacuation selon la revendication 1, **caractérisé en ce que** la face du fond (11) tournée vers l'espace intérieur est conique ou convexe, de sorte que les objets ou matières présents dans le contenant (10a,10b,10c,10d,10e) sortent du contenant (10) par gravité soit par le premier orifice ou par le deuxième orifice (41,43,45,47, 42,44,46,48).

3. Contenant à auto-évacuation selon la revendication 2, **caractérisé en ce que** la pointe du cône ou le point le plus haut du fond coïncide avec l'axe de rotation (X).

4. Contenant à auto-évacuation selon l'une des revendications précédentes, **caractérisé en ce que** le fond (11) est disposé en position inclinée, de sorte que les deux orifices (41,43,45,47, 42,44,46,48) sont situés dans la zone du point le plus bas du contenant (10a,10b,10c,10d,10e) et que les objets ou matières présents dans le contenant (10a,10b,10c,10d,10e) sortent du contenant (10a,10b,10c,10d,10e) par gravité soit par le premier orifice ou par le deuxième orifice (41,43,45,47, 42,44,46,48).

5. Contenant à auto-évacuation selon l'une des revendications précédentes, **caractérisé en ce que** la face inférieure de l'entretoise (12,12') s'appuie sur le fond (11).

6. Contenant à auto-évacuation selon l'une des revendications précédentes, **caractérisé en ce qu'**un balai ou des crins, qui se trouvent en contact avec le fond (11), sont disposés sur la face inférieur de l'entretoise (12,12') pour enlever les objets ou matières.

7. Contenant à auto-évacuation selon l'une des revendications précédentes, **caractérisé en ce que** l'entretoise (12, 12') peut simultanément faire office de barrière (12,12') qui empêche que les objets ou matières présents dans le contenant (10a,10b,10c,10d,10e) puissent participer à une pleine rotation du fond (11).

8. Contenant à auto-évacuation selon l'une des revendications précédentes, **caractérisé en ce que** l'entretoise (12,12') dépasse la paroi latérale (20) vers l'extérieur et s'introduit dans au moins l'un des orifices (47,48) et qu'une arête de la paroi latérale (20) tournée vers le premier orifice (47) fait office de première butée (23') et une arête de la paroi latérale (20) tournée vers le deuxième orifice (48) fait office de deuxième butée (24').

9. Contenant à auto-évacuation selon l'une des revendications précédentes, **caractérisé par** un nez d'arrêt (25) qui sert à la fois de première et de deuxième butée.

10. Contenant à auto-évacuation selon l'une des revendications précédentes, **caractérisé par** un commutateur servant à la commutation à distance du sens de rotation du fond (11).

11. Contenant à auto-évacuation selon l'une des revendications précédentes, **caractérisé en ce que** l'axe de pivotement coïncide avec l'axe de rotation (X).

12. Procédé pour évacuer un contenant (10a,10b,10c,10d,10e) sous forme de cylindre ou de bol comprenant
- une paroi latérale (20) entourant périphériquement de façon rotationnellement symétrique ou non l'espace intérieur du contenant (10a,10b,10c,10d,10e) et présentant une première et une deuxième butée (23,24, 23',24', 25) ainsi qu'un premier et un deuxième orifice (41,43,45,47, 42,44,46,48)
- ainsi qu'un fond (11) lequel, au moyen d'un entraînement, peut être mis en rotation par rapport à la paroi latérale (20) dans l'un ou l'autre des deux sens autour d'un axe de rotation (X) qui coïncide avec l'axe central de la paroi latérale (20),
avec utilisation d'une entretoise (12,12') disposée à l'intérieur du contenant (10a,10b,10c,10d,10e) et logée de façon pivotante autour d'un axe de pivotement soit par rapport au fond (11) soit par rapport à la paroi latérale (20),
dans lequel
- la déviation du pivotement de l'entretoise (12,12') par rapport à la paroi latérale (20) est limitée dans l'un des sens par la première butée (23,23',25) et dans le sens opposé par la deuxième butée (24,24',25), de sorte que l'entretoise (12,12'), lorsqu'elle s'appuie contre la première butée (23,23',25), se trouve dans une première position de butée et lorsqu'elle s'appuie contre la deuxième butée (24,24',25), se trouve dans une deuxième position de butée, l'entretoise (12,12') ne pouvant pivoter que dans un intervalle d'angle déterminé (α,β,χ,δ,ε) entre ces deux positions de butée,
- l'entretoise (12,12') est couplée au fond (11) par adhérence ou par friction, si bien que, lorsque le fond (11) tourne dans l'un des sens de rotation, l'entretoise (12,12') se déplace dans la première position de butée, et, lorsque le fond (11) tourne dans l'autre sens de rotation, elle se déplace dans la deuxième position de butée, et/ou l'entretoise (12,12') peut être déplacée de la première position de butée vers la deuxième position de butée et vice versa au moyen d'un entraînement séparé,
- et dans lequel l'entretoise (12,12') est conformée et les butées (23,24, 23',24', 25) sont disposées de telle manière que l'entretoise (12,12'), dans la première position de butée, bloque le premier orifice (41,43,45,47) et débloque le deuxième orifice (42,44,46,48), et dans la deuxième position de butée, débloque le premier orifice (41,43,45,47) et bloque le deuxième orifice (42,44,46,48),
le sens de rotation du fond (11) autour de l'axe de rotation (X)
- étant choisi de sorte que, en fonction du sens de rotation du fond (11), les objets ou matières présents dans le contenant (10a,10b,10c,10d,10e) peuvent sortir du contenant (10,10b,10c,10d,10e) uniquement par le premier orifice (41,43,45,47),
- ou étant choisi de sorte que, en fonction du sens de rotation du fond (11), les objets ou matières présents dans le contenant (10a,10b,10c,10d,10e) peuvent sortir du contenant (10,10b,10c,10d,10e) uniquement par le deuxième orifice (42,44,46,48).

13. Procédé selon la revendication 12, **caractérisé en ce que** le fond (11) utilisé est conique ou convexe, de sorte que les objets ou matières présents dans le contenant (10a,10b,10c,10d,10e) sortent du contenant (10) par gravité soit par le premier orifice ou par le deuxième orifice (41,43,45,47, 42,44,46,48).

14. Procédé selon la revendication 13, **caractérisé en ce qu'**on utilise un fond dont la pointe du cône ou le point le plus haut du fond coïncide avec l'axe de rotation (X).

15. Procédé selon l'une des revendication 12 à 14, **caractérisé en ce que** le fond (11) est disposé en position inclinée, de sorte que les deux orifices (41,43,45,47, 42,44,46,48) sont situés dans la zone du point le plus bas du contenant (10a,10b,10c,10d,10e) et que les objets ou matières présents dans le contenant (10a,10b,10c,10d,10e) sortent du contenant (10a,10b,10c,10d,10e) par gravité soit par le premier orifice ou par le deuxième orifice (41,43,45,47, 42,44,46,48).

16. Procédé selon l'une des revendications 12 '15, **caractérisé en ce qu'**on utilise une entretoise (12,12') dont la face inférieure s'appuie sur le fond (11).

17. Procédé selon la revendication 12, **caractérisé en ce qu'**on utilise une entretoise (12,12') dont la face inférieure est dotée d'un balai ou de crins qui se trouvent en contact avec le fond (11) et servent à enlever les objets ou matières.

18. Procédé selon l'une des revendications 12 à 17, **caractérisé en ce que** l'entretoise (12, 12') fait en même temps office de barrière (12,12') qui empêche que les objets ou matières présents dans le contenant (10a,10b,10c,10d,10e) puissent participer à une pleine rotation du fond (11).

19. Procédé selon l'une des revendications 12 à 18, **caractérisé en ce qu'**on utilise une entretoise (12') qui dépasse la paroi latérale (20) vers l'extérieur et s'introduit dans au moins l'un des orifices (47,48), une arête de la paroi latérale (20) tournée vers le premier orifice (47) faisant office de première butée (23') et une arête de la paroi latérale (20) tournée vers le deuxième orifice (48) faisant office de deuxième butée (24').

20. Procédé selon l'une des revendications 12 à 19, **caractérisé en ce qu'**on utilise un nez d'arrêt (25), qui sert à la fois de première et de deuxième butée.

21. Procédé selon l'une des revendications 12 à 20, **caractérisé en ce que** le sens de rotation du fond (11) est commuté au moyen d'un commutateur.

22. Procédé selon l'une des revendications 12 à 21, **caractérisé en ce que** l'axe de pivotement coïncide avec l'axe de rotation (X).
